# EUROPEAN PATENT APPLICATION

(11) **EP 3 495 831 A1**
(43) Date of publication of application: **12.06.2019**
(21) Application number: 18209580.2
(22) Date of filing: 30.11.2018
(51) Int. Cl.: G01R 33/24, G01R 33/36, G01R 33/34, G01R 33/565

(54) **COIL APPARATUS, MAGNETIC RESONANCE IMAGING APPARATUS, AND METHOD OF CONTROLLING THE COIL APPARATUS**

(30) Priority: 01.12.2017 KR 20170164252
(71) Applicant: Samsung Electronics Co., Ltd., Gyeonggi-do 16677 (KR)
(72) Inventor: VERGHESE, George, 16677 Gyeonggi-do (KR)
(74) Representative: D'Halleweyn, Nele Veerle Trees Gertrudis

(57) **Abstract**

A coil apparatus, including a radio frequency (RF) coil configured to transmit an RF transmission signal onto an object; a sensor configured to detect a static magnetic field formed in a magnetic resonance imaging (MRI) apparatus; a processor configured to determine a direction of the static magnetic field, determine an orientation direction of the coil apparatus with respect to the determined direction of the static magnetic field, and transmit a control signal for controlling a phase of an RF signal that is applied to the RF coil, based on the orientation direction of the coil apparatus; and a switch configured to change the phase of the RF signal that is applied to the RF coil, based on the control signal.

## Description

### BACKGROUND

### 1. Field

The disclosure relates to a coil apparatus, a Magnetic Resonance Imaging (MRI) apparatus, and a method of controlling the coil apparatus, and more particularly, to a technique for controlling a coil apparatus for transmitting a Radio Frequency (RF) signal onto an object and receiving an excited Magnetic Resonance (MR) signal from the object to normally operate corresponding to a direction of a magnetic field formed in a MRI apparatus.

### 2. Description of the Related Art

In general, a medical imaging apparatus is an apparatus for providing information about a patient as an image. Examples of a medical imaging apparatus are an X-ray apparatus, an ultrasonic diagnostic apparatus, a computer tomography (CT) scanner, a Magnetic Resonance Imaging (MRI) apparatus, etc.
an MRI apparatus holds an important position in the field of medical imaging diagnosis because image-taking conditions are relatively free and it can provide excellent contrast and various diagnosis information images for soft tissue.

MRI causes nuclear magnetic resonance (NMR) in the hydrogen atomic nuclei of the human body using a magnetic field harmless to humans as well as RF, which is non-ionizing radiation, to thus image the densities and physical or chemical characteristics of the atomic nuclei.

An MRI apparatus may include an RF transmitting coil for transmitting RF pulses, and an RF receiving coil for receiving electromagnetic waves, that is, Magnetic Resonance (MR) signals emitted from excited atomic nuclei.

Also, an MRI apparatus may receive MR signals excited in an object from a local coil apparatus including a plurality of RF receiving coils, as an external apparatus which assists the MRI apparatus and is independent from the MRI apparatus.

For the coils transmitting and receiving RF signals and the local coil apparatus to properly operate to receive excited MR signals from an object, the coils need to be installed in a correct direction with respect to a direction of a magnetic field formed in the MRI apparatus.

### SUMMARY

Therefore, it is an aspect of the present disclosure to provide a coil apparatus for determining a direction of a magnetic field formed in a Magnetic Resonance Imaging (MRI) apparatus, and changing a phase of an RF signal that is applied to an RF coil to thereby operate the RF coil normally in the magnetic field formed in the MRI apparatus regardless of a position of the coil apparatus, a MRI apparatus, and a method of controlling the coil apparatus.

Additional aspects of the disclosure will be set forth in part in the description which follows and, in part, will be obvious from the description, or may be learned by practice of the disclosure.

In accordance with an aspect of the present disclosure, there is provided a coil apparatus including a radio frequency (RF) coil configured to transmit an RF transmission signal onto an object; a sensor configured to detect a static magnetic field formed in a magnetic resonance imaging (MRI) apparatus; a processor configured to determine a direction of the static magnetic field, determine an orientation direction of the coil apparatus with respect to the determined direction of the static magnetic field, and transmit a control signal for controlling a phase of an RF signal that is applied to the RF coil, based on the orientation direction of the coil apparatus; and a switch configured to change the phase of the RF signal that is applied to the RF coil, based on the control signal.

Based on determining that the orientation direction of the coil apparatus is opposite to a predetermined direction with respect to the determined direction of the static magnetic field, the processor may be further configured to transmit a control signal for changing the phase of the RF signal, and based on determining that the orientation direction of the coil apparatus is the predetermined direction, the processor transmits a control signal for not changing the phase of the RF signal.

Based on determining that the orientation direction of the coil apparatus is opposite to a predetermined direction, the processor may be further configured to transmit a control signal for changing the phase of the RF signal that is transmitted by the RF coil by 90° .

The processor may be further configured to determine the orientation direction of the coil apparatus based on a rotation direction of a B1 field formed in a direction that is perpendicular to the static magnetic field by the RF signal that is applied to the RF coil.

Based on determining that that the rotation direction of the B1 field is opposite to a predetermined rotation direction, the processor may be further configured to determine that the orientation direction of the coil apparatus is opposite to the predetermined direction.

The sensor may be further configured to detect a hall voltage based on the static magnetic field formed in the MRI apparatus.

The processor may be further configured to determine the direction of the static magnetic field based on a polarity of the detected hall voltage, and the polarity of the hall voltage may change according to a direction of a potential difference generated based on the static magnetic field.

The switch may be further configured to change the phase of the RF signal by 90° according to the control signal, and to apply the RF signal to the RF coil.

The coil apparatus may further include a DC conductor configured to detect a hall voltage based on the static magnetic field formed in the MRI apparatus.

The RF coil may include an RF transmitting coil configured to transmit the RF transmission signal onto the object, and an RF receiving coil configured to receive an excited Magnetic Resonance (MR) signal from the object.

The RF coil may include a birdcage coil.

In accordance with an aspect of the present disclosure, there is provided a method of controlling a coil apparatus, including detecting a static magnetic field formed in a Magnetic Resonance Imaging (MRI) apparatus; determining a direction of the static magnetic field; determining an orientation direction of the coil apparatus with respect to the determined direction of the static magnetic field; transmitting a control signal for controlling a phase of a Radio Frequency (RF) signal that is applied to an RF coil, based on the orientation direction of the coil apparatus; and changing the phase of the RF signal that is applied to the RF coil, based on the control signal.

The transmitting of the control signal for controlling the phase of the RF signal that is applied to the RF coil may include transmitting a control signal for changing the phase of the RF signal based on determining that the orientation direction of the coil apparatus is opposite to a predetermined direction; and transmitting a control signal for not changing the phase of the RF signal based on determining that the orientation direction of the coil apparatus is the predetermined direction.

In accordance with an aspect of the present disclosure, there is provided a magnetic resonance imaging (MRI) apparatus including a static magnetic field generator configured to form a static magnetic field in an inner space of the MRI apparatus; a Radio Frequency (RF) coil configured to transmit an RF transmission signal onto an object; a magnetic field sensor configured to detect the static magnetic field; a controller configured to determine a direction of the static magnetic field based on the detected static magnetic field, determine an orientation direction of the RF coil with respect to the determined direction of the static magnetic field, and transmit a control signal for controlling a phase of an RF signal that is applied to the RF coil, based on the orientation direction of the RF coil; and a switch configured to change the phase of the RF signal that is applied to the RF coil, based on the control signal.

The RF coil may include an RF transmitting coil configured to transmit the RF transmission signal onto the object, and an RF receiving coil configured to receive an excited Magnetic Resonance (MR) signal from the object.

Based on determining that an orientation direction of the RF transmitting coil is opposite to a predetermined direction with respect to the determined direction of the static magnetic field, the controller may be further configured to transmit a control signal for changing the phase of the RF signal, and based on determining that the orientation direction of the RF transmitting coil is the predetermined direction, the controller may be further configured to transmit a control signal for not changing the phase of the RF signal.

Based on determining that an orientation direction of the RF transmitting coil is opposite to a predetermined direction with respect to the determined direction of the static magnetic field, the controller may be further configured to transmit a control signal for changing the phase of the RF signal that is applied to the RF coil by 90°.

The magnetic field sensor may be further configured to detect a hall voltage based on the static magnetic field formed in the MRI apparatus.

The controller may be further configured to determine the direction of the static magnetic field based on a polarity of the detected hall voltage, and the polarity of the hall voltage may change according to a direction of a potential difference generated based on the static magnetic field.

The switch may be configured to change the phase of the RF signal by 90° according to the control signal, and applies the RF signal to the RF transmitting coil.

In accordance with an aspect of the present disclosure, there is provided a coil apparatus including a radio frequency (RF) coil configured to transmit an RF transmission signal onto an object; a sensor configured to detect a static magnetic field formed in a magnetic resonance imaging (MRI) apparatus; a processor configured to determine a direction of the static magnetic field, determine a relationship between an orientation direction of the coil apparatus and the determined direction of the static magnetic field, and a switch configured to change a phase of an RF signal that is applied to the RF coil, based on the determined relationship.

Based on the processor determining that the orientation direction of the coil apparatus is opposite to the determined direction of the static magnetic field, the switch may be further configured to change the phase of the RF signal.

### BRIEF DESCRIPTION OF THE DRAWINGS

The above and other aspects, features, and advantages of certain embodiment of the present disclosure will be more apparent from the following description taken in conjunction with the accompanying drawings, in which:
FIG. 1 is a schematic view of a Magnetic Resonance Imaging (MRI) apparatus according to an embodiment;
FIG. 2, FIG. 3, and FIG. 4 show outer appearances of coil apparatuses according to embodiments;
FIG. 5 is a conceptual view showing directions of static magnetic fields formed in a MRI apparatus and a coil apparatus positioned in correspondence to the directions of the static magnetic fields, according to an embodiment;
FIG. 6 shows a B1 field formed in a coil apparatus in a direction that is perpendicular to a static magnetic field formed in a MRI apparatus, according to an embodiment;
FIG. 7 is a control block diagram of a coil apparatus according to an embodiment;
FIG. 8 shows a sensor according to an embodiment that detects a hall voltage based on a static magnetic field;
FIG. 9 is a view for describing a case of not changing a phase of a Radio Frequency (RF) signal that is applied to an RF coil, according to an embodiment;
FIG. 10 is a view for describing a case of changing a phase of a RF signal that is applied to an RF coil, according to an embodiment;
FIG. 11 and FIG. 12 are flow charts illustrating a coil apparatus controlling method according to an embodiment;
FIG. 13 is a block diagram showing a control flow of a MRI apparatus according to an embodiment; and
FIG. 14 shows a structure of a scanner according to an embodiment.

### DETAILED DESCRIPTION

Hereinafter, like reference numerals will refer to like components throughout this specification. This specification does not describe all components of the embodiments, and general information in the technical field to which the present disclosure belongs or overlapping information between the embodiments will not be described. As used herein, the terms "portion", "part, "module, "member" or "block" may be implemented as software or hardware, and according to embodiments, a plurality of "portions", "parts, "modules, "members" or "blocks" may be implemented as a single component, or a single "portion", "part, "module, "member" or "block" may include a plurality of components.

Also, it will be understood that when a certain part "includes" a certain component, the part does not exclude another component but can further include another component, unless the context clearly dictates otherwise.

Also, it will be understood that, although the terms first, second, etc. may be used herein to describe various components, these components should not be limited by these terms. These terms are only used to distinguish one component from another.

Also, it is to be understood that the singular forms "a," "an," and "the" include plural referents unless the context clearly dictates otherwise.

In this specification, the term "image" may mean multi-dimensional data configured with discrete image elements, for example, pixels in a 2-Dimensional (2D) image and voxels in a 3-Dimensional (3D) image. For example, an image may include a medical image of an object, acquired by an X-ray imaging apparatus, a Computed Tomography (CT) apparatus, a Magnetic Resonance Imaging (MRI) apparatus, an ultrasonic apparatus, and other medical imaging systems.

In this specification, the term "object" may be a human, an animal, or a part of a human or an animal. For example, the object may include an organ, such as skin, liver, heart, uterus, brain, breasts, abdomen, etc., or blood vessels. Also, the term "object" may be a phantom. The phantom means a material having a volume that is very close to the density and effective atomic number of a living thing, and may include a spherical phantom having similar properties to the human body.

In this specification, the term "user" may be a medical specialist including a doctor, a nurse, a medical technologist, and a radiological technologist, and may also be an engineer who repairs medical equipment. However, the user is not limited to the above-mentioned persons.

A Magnetic Resonance Imaging (MRI) system is a system for acquiring a Magnetic Resonance (MR) signal and reconstructing the MR signal as an image. The MR signal is a Radio Frequency (RF) signal emitted from an object.

In the MRI system, a main magnet forms a static magnetic field, and the MRI system aligns a magnetic dipole moment direction of specific atomic nuclei of an object located in the static magnetic field in the direction of the static magnetic field. A gradient magnetic field coil applies a gradient signal to the static magnetic field to form a gradient magnetic field, thereby inducing different resonance frequencies for different regions of the object.

A RF coil irradiates an RF signal corresponding to a resonance frequency of a target region from which an image will be acquired. Also, as the gradient magnetic field is formed, the RF coil receives MR signals of different resonance frequencies emitted from the object's various regions. Then, the MRI system acquires an image from the MR signals using an image reconstruction technique.

Hereinafter, the operation principle and embodiments will be described with reference to the accompanying drawings.

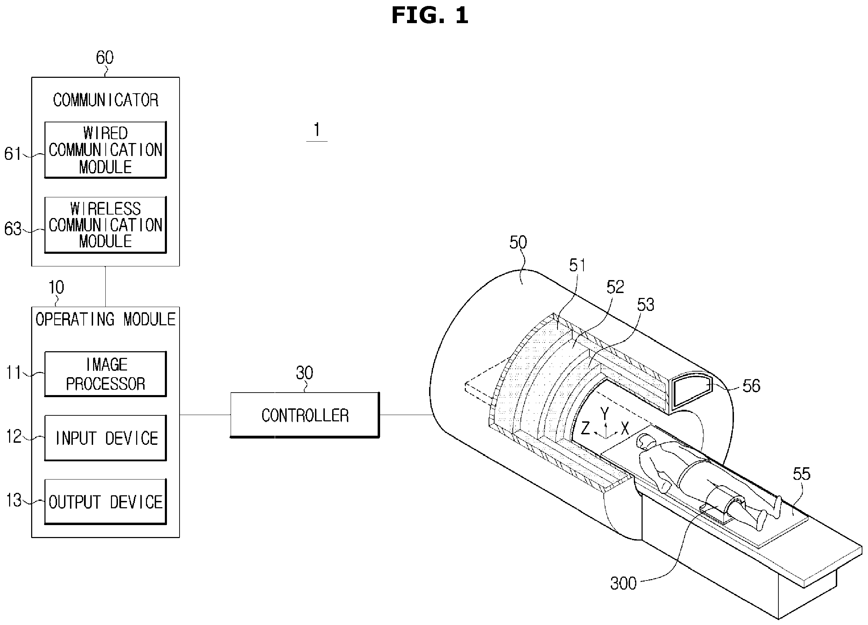

FIG. 1 is a schematic view of a MRI apparatus.

Referring to FIG. 1, a MRI apparatus 1 may include an operating module 10, a controller 30, and a scanner 50. Herein, the controller 30 may be, as shown in FIG. 1, implemented independently. In an embodiment, the controller 30 may be divided to a plurality of components to be respectively included in individual components of the MRI apparatus 1. Hereinafter, the individual components of the MRI apparatus 1 will be described in detail.

The scanner 50 may be implemented in a shape, for example, a bore shape, whose inside space is empty and into which an object may be inserted. In the inside space of the scanner 50, a static magnetic field and a gradient magnetic field may be formed, and a RF signal may be irradiated.

The scanner 50 may include a static magnetic field generator 51, a gradient magnetic field generator 52, an RF coil portion 53, a transfer table 55, and a display 56. The static magnetic field generator 51 may form a static magnetic field for aligning a magnetic dipole moment direction of atomic nuclei included in the object in a direction of the static magnetic field. The static magnetic field generator 51 may be implemented as a permanent magnet or a superconductive magnet using a cooling coil.

The gradient magnetic field generator 52 may be connected to the controller 30. The gradient magnetic field generator 52 may apply a gradient to the static magnetic field according to a control signal received from the controller 30 to form a gradient magnetic field. The gradient magnetic field generator 52 may include X, Y, and Z coils for forming gradient magnetic fields in X-, Y-, and Z-axis directions that are orthogonal to each other, and generate a gradient signal in correspondence to a scanning location so as to induce different resonance frequencies for the object's different parts.

The RF coil portion 53 may be connected to the controller 30. The RF coil portion 53 may irradiate an RF signal onto the object according to a control signal received from the controller 30, and receive a MR signal emitted from the object. The RF coil portion 53 may transmit an RF signal of the same frequency as that of precession to the object toward atomic nuclei that perform the precession, then stop transmitting the RF signal, and receive a MR signal emitted from the object.

The RF coil portion 53 may be implemented as an RF transmitting coil for generating electronic waves having a radio frequency corresponding to the kind of atomic nuclei, and an RF receiving coil for receiving electronic waves emitted from the atomic nuclei, or the RF coil portion 53 may be implemented as a single RF transmitting and receiving coil having both a transmitting function and a receiving function.

The RF transmitting coil may be implemented as a whole-volume coil for transmitting an RF pulse to the entire of the object, and the RF receiving coil may also be implemented as a whole-volume coil for receiving an excited MR signal from the entire of the object. The whole-volume coil may also be referred to as a body coil.

Also, the RF receiving coil may be provided in an external apparatus, hereinafter referred to as a coil apparatus 300, that may be independent from the scanner 50, and mounted on the object. The coil apparatus 300 may be connected to the scanner 50, the controller 30, and the operating module 10 through a signal transceiver such as a cable, and transmit data about a MR signal generated from atomic nuclei to an image processor 11. The coil apparatus 300 may be implemented as a dedicated coil, such as a head coil, a spine coil, a torso coil, and a knee coil, according to a region that is to be scanned or a region on which it is mounted. The coil apparatus 300 shown in FIG. 1 may be implemented as a knee coil.

The coil apparatus 300 may function as both the RF transmitting coil and the RF receiving coil, or may function as the RF receiving coil.

On at least one of an inner or an outer surface of the scanner 50, a display 56 may be provided. The display 56 may be controlled by the controller 30, and provide a user or an object with information related to medical image scanning.

Also, the scanner 50 may include an object monitor for acquiring monitoring information about the object's state and transmitting the monitoring information. For example, the object monitor may acquire monitoring information about an object from a camera for photographing a motion, a location, etc. of the object, a spirometer for measuring a breath of the object, an electrocardiogram (ECG) system for measuring an electrocardiogram of the object, or a thermometer for measuring a temperature of the object, and transfer the monitoring information to the controller 30. Accordingly, the controller 30 may control an operation of the scanner 50 using the monitoring information about the object. Hereinafter, the controller 30 will be described.

The controller 30 may control overall operations of the scanner 50.

The controller 30 may control sequences of signals formed in the inside of the scanner 50. The controller 30 may control the gradient magnetic field generator 52 and the RF coil portion 53 according to a pulse sequence received from the operating module 10 or a designed pulse sequence.

The pulse sequence may include all information required for controlling the gradient magnetic field generator 52 and the RF coil portion 53. For example, the pulse sequence may include information about strength of a pulse signal that is applied to the gradient magnetic field generator 52, a time for which the pulse signal is applied, a timing at which the pulse signal is applied, etc.

The controller 30 may control a waveform generator for generating a gradient waveform, that is, a current pulse according to a pulse sequence, and a gradient amplifier for amplifying the current pulse and transferring the amplified current pulse to the gradient magnetic field generator 52, thereby controlling the gradient magnetic field generator 52 to form a gradient magnetic field.

The controller 30 may control operations of the RF coil portion 53. For example, the controller 30 may supply an RF pulse of a resonance frequency to the RF coil portion 53 to irradiate an RF signal, and receive a MR signal received by the RF coil portion 53. At this time, the controller 30 may control an operation of a switch, for example, a T/R switch, capable of adjusting a transmitting or receiving direction, through a control signal, to irradiate an RF signal and to receive a MR signal according to an operation mode. The switch will be described in detail, later.

The controller 30 may control a movement of the transfer table 55 on which the object is placed. Before scanning is performed, the controller 30 may move the transfer table 55 in advance to correspond to the object's region to be scanned.

The controller 30 may control the display 56. For example, the controller 30 may control an on/off function of the display 56 or a screen to be displayed on the display 56, through a control signal.

The controller 30 may control the coil apparatus 300. For example, the controller 30 may control an operation of a switch, for example, an on/off switch, for receiving or not receiving a MR signal, through a control signal, thereby receiving or not receiving a MR signal of the coil apparatus 300 according to an operation mode.

The controller 30 may be implemented with a memory that stores algorithms for controlling operations of components in the MRI apparatus 1, and data of program formats, and a processor that performs the above-described operations using the data stored in the memory. The memory and the processor may be implemented as separate chips. In an embodiment, the memory and the processor may be implemented as a single chip.

The operating module 10 may control overall operations of the MRI apparatus 1. The operating module 10 may include the image processor 11, an input device 12, and an output device 13.

The image processor 11 may store a MR signal received from the controller 30 using the memory, and apply an image reconstruction method to the MR signal using the processor to thereby create an image about the object from the MR signal.

For example, the image processor 11 may fill digital data in a k-space, which may also referred to as a Fourier space or a frequency space, of the memory, and when k-space data is completed, the image processor 11 may apply various image reconstruction methods, for example, Inverse Fourier Transform, to the k-space data through the processor to reconstruct the k-space data as an image.

Also, various signal processes which the image processor 11 applies to the MR signal may be performed in parallel. For example, the image processor 11 may perform signal-processing in parallel on a plurality of MR signals received by a multi-channel RF coil to reconstruct the MR signals as an image. Meanwhile, the image processor 11 may store the reconstructed image in the memory, or the controller 30 may store the reconstructed image in an external server through a communicator 60, which will be described later.

The input device 12 may receive a control command for overall operations of the MRI apparatus 1 from a user. For example, the input device 12 may receive object information, parameter information, a scan condition, information about a pulse sequence, etc. from the user. The input device 12 may be implemented as a keyboard, a mouse, a trackball, a voice recognizer, a gesture recognizer, a touch screen, etc.

The output device 13 may output an image created by the image processor 11. Also, the output device 13 may output a user interface (UI) configured to receive a control command for the MRI apparatus 1 from the user. The output device 13 may be implemented as a speaker, a printer, a display, etc., and the display may include the display 56 disposed on at least one of the outer or inner surface of the scanner 50. An embodiment described below relates to a case in which the output device 13 is implemented as a display, however, embodiments are not limited to this.

The display may be a Cathode Ray Tube (CRT), a Digital Light Processing (DLP) panel, a Plasma Display Panel (PDP), a Liquid Crystal Display (LCD) panel, an Electro Luminescence (EL) panel, an Electrophoretic Display (EPD) panel, an Electrochromic Display (ECD) panel, a Light Emitting Diode (LED) display, or an Organic Light Emitting Diode (OLED) panel, although it is not limited to these.

Meanwhile, in FIG. 1, the operating module 10 and the controller 30 are shown as separated components, however, the operating module 10 and the controller 30 may be included in a single device. Also, processes that are performed by the operating module 10 and the controller 30 may be performed by another device. For example, the image processor 11 may convert a MR signal received from the controller 30 to a digital signal, or the controller 30 may itself convert a MR signal to a digital signal.

Also, at least one component may be added or deleted in correspondence to the performances of the components of the MRI apparatus 1 shown in FIG. 1. Also, it will be easily understood to one of ordinary skill in the art that the relative positions of the components may be changed in correspondence to the performance or structure of the MRI apparatus 1.

Meanwhile, each of the components shown in FIG. 1 may be a software component and/or a hardware component, such as a Field Programmable Gate Array (FPGA) or an Application Specific Integrated Circuit (ASIC).

Hereinafter, the coil apparatus 300 according to an embodiment will be described. The coil apparatus 300 which will be described below may include an RF transmitting coil for irradiating an RF signal onto an object, and an RF receiving coil for receiving an excited MR signal from the object, wherein the RF receiving coil is assumed to receive an excited MR signal from a region of the object.

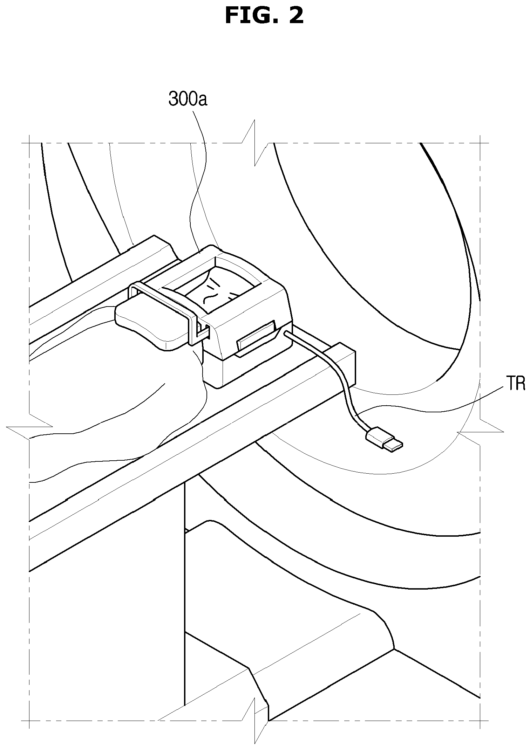

FIGS. 2 to 4 show outer appearances of coil apparatuses according to various embodiments.

As shown in FIG. 2, the coil apparatus 300 may be implemented as a coil apparatus 300a, which may be a head coil apparatus for scanning an object's head and receiving an excited MR signal from the object's head.

In the coil apparatus 300a, a plurality of RF transmitting coils and a plurality of RF receiving coils may be installed, and the plurality of RF transmitting coils may irradiate an RF signal onto the object's head. The plurality of RF receiving coils may receive an echo signal, that is, a MR signal generated from the object's head, and transmit data about the MR signal to the image processor 11 of the MRI apparatus 1 through a signal transceiver TR such as a cable to acquire an MR image about the object's head.

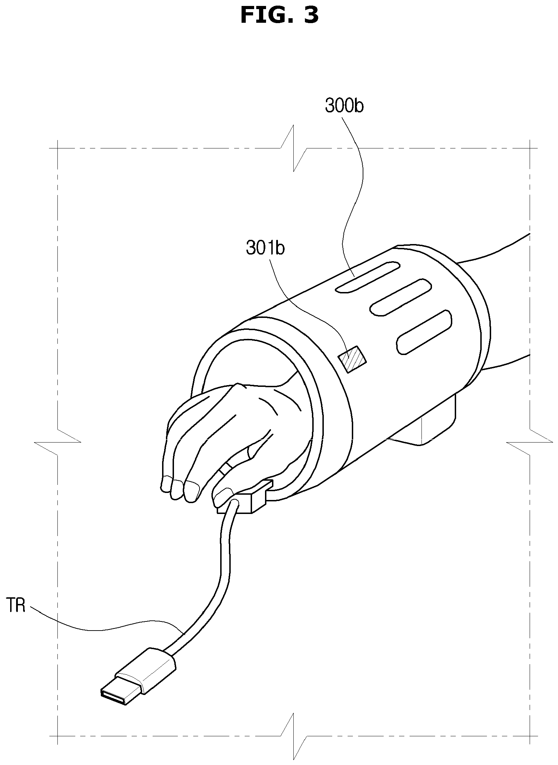

Also, as shown in FIG. 3, the coil apparatus 300 may be implemented as a coil apparatus 300b for scanning an object's localized region and receiving an excited MR signal from the object's localized region. Herein, the object's localized region may be one of the object's various regions, such as an arm, a leg, etc.

In the coil apparatus 300b, likewise, a plurality of RF transmitting coils and a plurality of RF receiving coils may be installed, and the plurality of RF transmitting coils may irradiate an RF signal onto the object's localized region. The plurality of RF receiving coils may receive an echo signal, that is, a MR signal generated from the object's localized region to acquire a MR image about the object's localized region. The coil apparatus 300b may also include a hall sensor 301b, which may correspond to hall sensor 301c described in greater detail below.

When the coil apparatus 300 is connected to the MRI apparatus 1 through a signal transceiver TR such as a cable, the RF receiving coils included in the coil apparatus 300 may be electrically connected to the scanner 50, the controller 30, and the image processor 11 of the MRI apparatus 1.

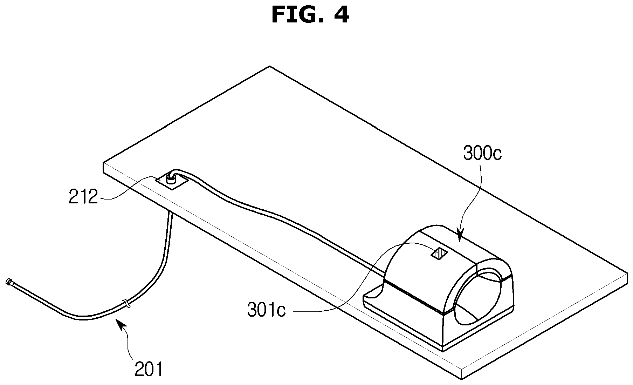

As shown in FIG. 4, the coil apparatus 300 may be implemented as a coil apparatus 300c, which may be a knee coil apparatus for scanning an object's knee and receiving an excited MR signal from the object's knee.

In the coil apparatus 300c, likewise, a plurality of RF transmitting coils and a plurality of RF receiving coils may be installed, and the plurality of RF transmitting coils may irradiate an RF signal onto the object's knee. The plurality of RF receiving coils may receive an echo signal, that is, a MR signal generated from the object's knee, and transmit data about the MR signal to the image processor 11 of the MRI apparatus 1 through a signal transceiver TR such as a cable to thereby acquire a MR image about the object's knee.

Referring to FIG. 4, the coil apparatus 300c may be manufactured in a circular shape to scan an object's knee, and have a cavity into which the object's knee is inserted.

Even after the object's knee is inserted into and fixed at the coil apparatus 300c, the direction of the coil apparatus 300c may change due to the object's movement. To prevent the problem, the coil apparatus 300c may include a hall sensor 301c. The MRI apparatus 1 may determine whether the coil apparatus 300c is properly fixed or whether a direction in which the coil apparatus 300c is coupled is a proper direction, based on a detection value transferred from the hall sensor 301c. The hall sensor 301c may be installed in the inside or outside of the coil apparatus 300c, although not limited thereto.

Also, in embodiments, the coil apparatus 300 may be implemented as a torso coil apparatus for scanning an object's chest or abdomen and receiving an excited MR signal from the object's chest or abdomen.

The above-described coil apparatus 300 may include a birdcage coil, a surface foil, and a TEM coil, according to the structure of coils.

The coil apparatus 300 shown in FIGS. 2 to 4 may include a birdcage coil. The coil apparatus 300 may be influenced by a direction of a magnetic field formed in the MRI apparatus 1, and in the case of a birdcage coil, when an alignment of the coil apparatus 300 does not correspond to a direction of a magnetic field formed in the MRI apparatus 1, the coil apparatus 300 may not operate properly.

That is, when an alignment of the coil apparatus 300 is different from a predetermined direction with respect to a direction of a magnetic field formed in the MRI apparatus 1, the quality of a MR image acquired by the coil apparatus 300 may deteriorate. Accordingly, it may be necessary to readjust the position of the coil apparatus 300.

Hereinafter, an example in which the coil apparatus 300 according to an embodiment is the coil apparatus 300c shown in FIG. 4 will be described. As discussed above, coil apparatus 300c may be, for example, a knee coil apparatus. However, the type of the coil apparatus 300 to which an embodiment is applied is not limited.

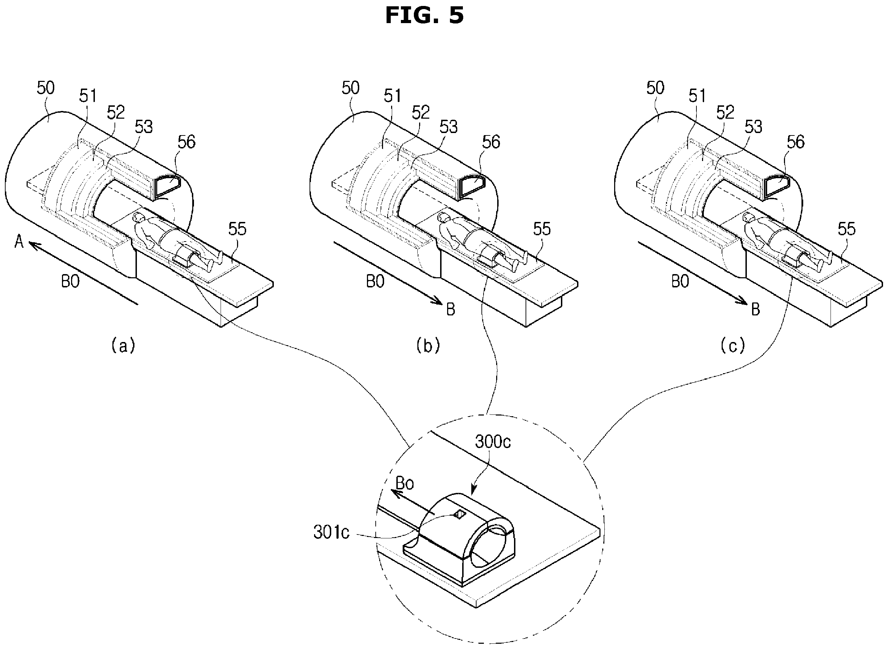

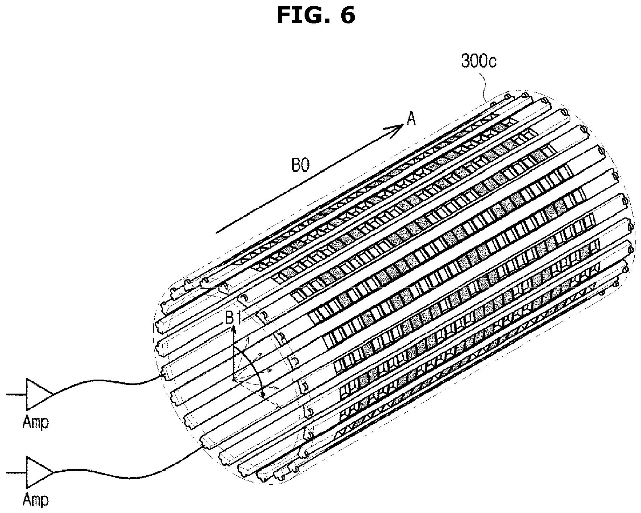

FIG. 5 is a conceptual view showing directions of static magnetic fields formed in a MRI apparatus and a coil apparatus positioned in correspondence to the directions of the static magnetic fields, according to embodiments. FIG. 6 shows a B1 field formed in a coil apparatus in a direction that is perpendicular to a static magnetic field formed in a MRI apparatus, according to an embodiment.

Referring to FIG. 5, the MRI apparatus 1 may form a static magnetic field by the static magnetic field generator 51, as described above. The static magnetic field is also referred to as a B0 field.

The MRI apparatus 1 may form a static magnetic field by a main magnet, and a direction of the static magnetic field may depend on the polarity of the main magnet. As described above, the coil apparatus 300 may be influenced by a direction of a magnetic field formed in the MRI apparatus 1, and when an alignment of the coil apparatus 300 does not correspond to a direction of a magnetic field formed in the MRI apparatus 1, the coil apparatus 300 may not operate properly.

That is, a directivity of the coil apparatus 300 with respect to a static magnetic field formed by the MRI apparatus 1 may be important to the operation of the coil apparatus 300 and the MRI apparatus 1.

In the MRI apparatus 1 shown in (a) of FIG. 5, a direction of a static magnetic field (B0 field) is an A direction, and in the MRI apparatus 1 shown in (b) and (c) of FIG. 5, a direction of a static magnetic field (B0 field) is a B direction.

In one example, a case in which the coil apparatus 300c operates properly when a direction of a static magnetic field formed in the MRI apparatus 1 is the A direction is assumed. In this example, when the coil apparatus 300c is disposed in the MRI apparatus 1 shown in (a) of FIG. 5 in the state in which it is positioned at a fixed location in a direction shown in FIG. 5, the coil apparatus 300c may operate properly to acquire a MR signal.

Meanwhile, because a direction of a static magnetic field formed in the MRI apparatus 1 shown in (b) and (c) of FIG. 5 is the B direction that is opposite to the A direction, the coil apparatus 300c may not operate properly when the coil apparatus 300c is disposed in the MRI apparatus 1 shown in (b) and (c) of FIG. 5 in the state in which the coil apparatus 300c is positioned at the fixed location in the direction shown in FIG. 5.

FIG. 6 is a perspective view showing an internal structure of the coil apparatus 300c shown in FIG. 5. As discussed above, coil apparatus 300c may be an example of coil apparatus 300.

When an RF signal is applied to the coil apparatus 300c, a RF field may be formed in a direction that is perpendicular to a static magnetic field B0 formed in the MRI apparatus 1 in the inside of the cavity of the coil apparatus 300c. That is, the RF field formed in the direction that is perpendicular to the static magnetic field B0 may be a B1 field, and the B1 field may rotate by the RF signal applied to the coil apparatus 300c, as shown in FIG. 6.

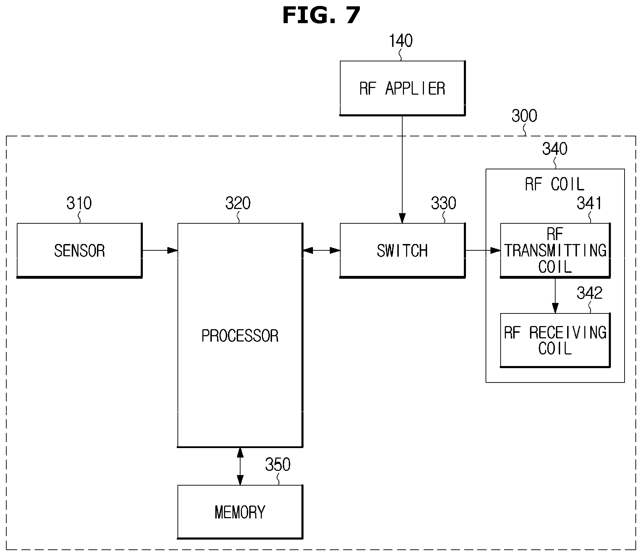

The RF signal applied to the coil apparatus 300c may be applied to the coil apparatus 300c through an RF applier 140 (as shown, for example, in FIG. 7) of the MRI apparatus 1. At this time, a plurality of RF signals may be applied from the MRI apparatus 1 to the coil apparatus 300c with phase differences of 90°.

The rotation direction of the B1 field may change according to a phase of an RF signal applied to the coil apparatus 300c. The quality of a MR image according to a MR signal may vary depending on the rotation direction of the B1 field.

For example, as shown in FIG. 6, when the direction of the static magnetic field B0 formed in the MRI apparatus 1 is the A direction, the coil apparatus 300c may need to be aligned in a predetermined direction with respect to the A direction of the static magnetic field B0, and when the coil apparatus 300c is positioned in the predetermined direction, a B1 field formed in the knee coil apparatus 300 may rotate in a direction shown in FIG. 6 to acquire a MR image.

However, when the coil apparatus 300c is positioned in a direction that is opposite to the predetermined direction with respect to the direction of the static magnetic field B1, the B1 field formed in the coil apparatus 300c may rotate in a direction that is opposite to the direction shown in FIG. 6. Accordingly, no MR image may be acquired, or the quality of a MR image may deteriorate.

A direction in which the B1 field rotates with respect to the direction of the static magnetic field B0 to acquire a MR image may change by cases.

Accordingly, when the alignment direction of the coil apparatus 300c is opposite to the predetermined direction with respect to the direction of the static magnetic field B0, it may be necessary to change the position of the coil apparatus 300c or to change a wiring of a cable connected to the coil apparatus 300c and transmitting an RF signal to change the phase of an RF signal that is applied to the coil apparatus 300c. However, changing the position of the coil apparatus 300c or changing the wiring of the cable may require additional time and cost.

In the coil apparatus according to an embodiment and the control method thereof, by determining a direction of a static magnetic field formed in the MRI apparatus 1 and changing a phase of an RF signal that is applied to the coil apparatus 300c, the coil apparatus 300c may operate normally in a magnetic field formed in the MRI apparatus 1 regardless of its position.

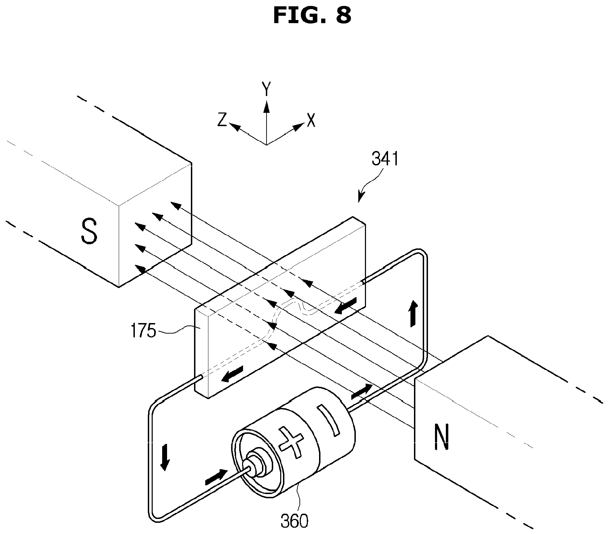

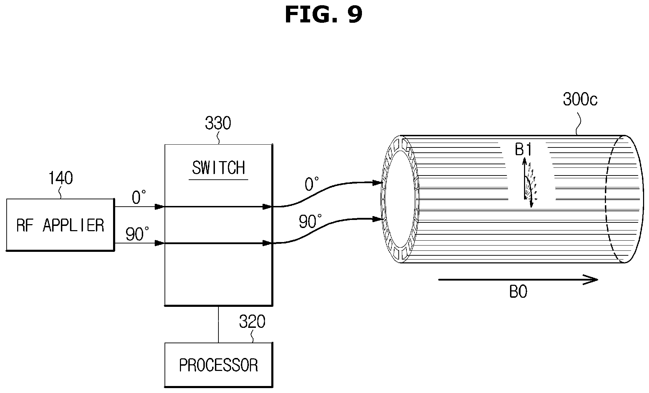

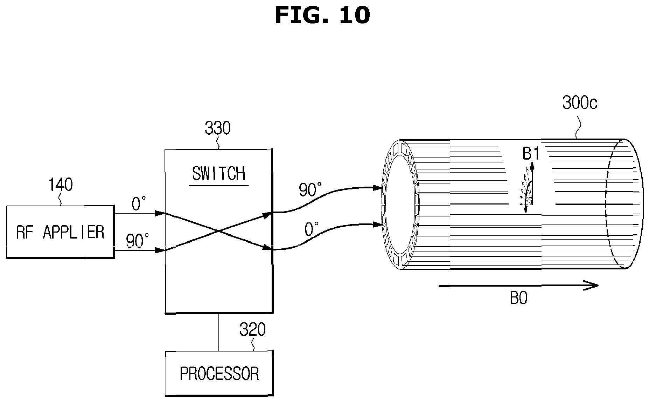

FIG. 7 is a control block diagram of a coil apparatus according to an embodiment. FIG. 8 shows a sensor according to an embodiment that detects a hall voltage based on a static magnetic field. FIG. 9 is a view for describing a case of not changing a phase of a RF signal that is applied to an RF coil, according to an embodiment. FIG. 10 is a view for describing a case of changing a phase of a RF signal that is applied to an RF coil, according to an embodiment.

Referring to FIG. 7, the coil apparatus 300 according to an embodiment may include a sensor 310 for detecting a hall voltage based on a static magnetic field, a processor 320 for controlling components of the coil apparatus 300, a switch 330 for changing a phase of an RF signal that is applied to an RF coil 340, the RF coil 340 for irradiating an RF signal onto an object and receiving an excited MR signal from the object, and a memory 350 for storing data related to operations of the coil apparatus 300.

The sensor 310 may detect a hall voltage based on a static magnetic field formed in the MRI apparatus 1, and transfer the hall voltage to the processor 320. The sensor 310 may be, as an example of a magnetic field sensor, a Hall effect sensor that detects a magnetic field based on the Hall effect.

The sensor 310 may be attached on the inside or outside surface of the coil apparatus 300. That is, the sensor 310 may be attached on an RF transmitting coil 341 or an RF receiving coil 342 of the RF coil 340, or may be an embedded type. Hereinafter, for convenience of description, the sensor 310 is assumed to be a hall sensor attached on the RF transmitting coil 341.

When the coil apparatus 300 including the RF transmitting coil 341 enters the inside of a gantry of the MRI apparatus 1, the sensor 310 may detect a hall voltage value caused by a static magnetic field formed in the inside of the gantry, and transfer the hall voltage value to the processor 320. Also, even when the coil apparatus 300 does not enter the inside of the gantry, the sensor 310 may detect a hall voltage caused by a static magnetic field formed in the MRI apparatus 1.

The processor 320 may determine a direction of the static magnetic field formed in the MRI apparatus 1 based on the hall voltage detected by the sensor 310.

The Hall effect may refer to a phenomenon in which a potential difference is generated. More specifically, the Hall effect may refer to a phenomenon in which, when a magnetic field is formed perpendicular to a direction of current flowing in a conductor, a potential difference (an electric field) is generated in the conductor.

In FIG. 8, a plurality of arrows connecting the N pole of a magnet with the S pole may form a magnetic field. The N pole and the S pole may correspond to the static magnetic field generator 51 of the MRI apparatus 1, and the plurality of arrows connecting the N pole with the S pole may indicate a static magnetic field.

When the sensor 310 receives power from a power source 360, current may flow from a negative (-) pole of the power source 360 to a positive (+) pole of the power source 360. That is, current may flow in an x-axis direction of FIG. 8. At this time, a potential difference may be generated in a y-axis direction in a conductor 175 through which the current flows.

The sensor 310 may detect the potential difference generated in the y-axis direction, that is, a hall voltage, wherein the hall voltage may change by a magnetic field. Accordingly, the sensor 310 may detect an intensity of an external magnetic field, and also may be used to detect direct current or alternating current through the intensity of the external magnetic field. Also, the sensor 310 may be applied to detect a rotation of a rotating body.

A value detected by the sensor 310 provided in the RF transmitting coil 341 may be used to determine a position of the coil apparatus 300 with respect to a direction of a static magnetic field, that is, a directivity of the coil apparatus 300.

The sensor 310 may be included in the coil apparatus 300 together with various other components, and the position and number of the sensor 310 are not limited.

More specifically, the processor 320 may determine a direction of a static magnetic field formed in the MRI apparatus 1, based on the hall voltage detected by the sensor 310. That is, because a polarity of a hall voltage detected by the sensor 310 changes according to a direction of a potential difference generated based on a static magnetic field, as described above, the processor 320 may determine a direction of a static magnetic field according to a direction of a potential difference causing a polarity of a hall voltage.

The coil apparatus 300 according to an embodiment may determine a direction of a static magnetic field based on a hall voltage detected by the sensor 310. However, the coil apparatus 300 may determine a direction of a static magnetic field based on a hall voltage detected by a DC conductor included in the coil apparatus 300, separately from the sensor 310. That is, the coil apparatus 300 may include a DC conductor therein, and the DC conductor may detect a hall voltage generated by a static magnetic field, and transfer the hall voltage to the processor 320.

The processor 320 may determine a direction of a static magnetic field formed in the MRI apparatus 1 based on a hall voltage detected by the sensor 310, and determine whether the coil apparatus 300 is positioned in a predetermined direction with respect to the determined direction of the static magnetic field.

That is, the processor 320 may determine whether the coil apparatus 300 is positioned in a predetermined direction with respect to a rotation direction of a B1 field formed in a direction that is perpendicular to a static magnetic field B0 by a RF signal that is applied to the RF coil 340.

As described above with reference to FIGS. 5 and 6, the rotation direction of the B1 field may change according to a phase of a RF signal that is applied to the RF coil 340, and when the coil apparatus 300 is positioned in the predetermined direction with respect to the direction of the static magnetic field B0, a MR image acquired by the rotating B1 field may have high quality.

Accordingly, the processor 320 may determine whether the coil apparatus 300 is positioned in the predetermined direction with respect to the direction of the static magnetic field B0 formed in the MRI apparatus 1, based on the rotation direction of the B1 field of the coil apparatus 300.

That is, the processor 320 may determine whether a rotation direction of a B1 field is a predetermined rotation direction with respect to the direction of the static magnetic field B0, and when the rotation direction of the B1 field is opposite to the predetermined rotation direction, the processor 320 may determine that the coil apparatus 300 is positioned in a direction that is opposite to the predetermined direction with respect to the direction of the static magnetic field B0.

Referring to FIGS. 9 and 10, when static magnetic fields B0 are formed in the same direction in the MRI apparatus 1, a B1 field formed in the coil apparatus 300c of FIG. 9 may rotate in a clockwise direction, and a B1 field formed in the coil apparatus 300c of FIG. 10 may rotate in a counterclockwise direction.

The processor 320 may determine whether coil apparatus 300c is positioned in a predetermined direction with respect to the direction of the static magnetic field B0 among the rotation direction of the B1 field formed in the coil apparatus 300c of FIG. 9 and the rotation direction of the B1 field formed in the coil apparatus 300c of FIG. 10, based on a hall voltage detected by the sensor 310.

The memory 350 may store data about a proper direction of the coil apparatus 300 with respect to a direction of a static magnetic field B0 formed in the MRI apparatus 1, and the processor 320 may determine whether the coil apparatus 300 is positioned in a predetermined direction, that is, the proper direction, based on the data stored in the memory 350.

The memory 350 may store data related to operations and control of the coil apparatus 300.

The memory 350 may be implemented as at least one of a non-volatile memory device, for example, a cache, Read Only Memory (ROM), Programmable ROM (PROM), Erasable Programmable ROM (EPROM), Electrically Erasable Programmable ROM (EEPROM), and flash memory, a volatile memory device, for example, Random Access Memory (RAM), or a storage medium, such as Hard Disk Drive (HDD) and Compact Disc Read Only Memory (CD-ROM), although not limited to these. The memory 350 may be implemented as a separate chip from the processor 320 described above, or the memory 350 and the processor 320 may be integrated into a single chip.

When the processor 320 determines that the coil apparatus 300 is positioned in a direction that is opposite to the predetermined direction with respect to the direction of the static magnetic field B0, the processor 320 may transmit a control signal for changing a phase of an RF signal that is applied to the RF coil 340.

That is, when the coil apparatus 300 is positioned in the direction that is opposite to the predetermined direction with respect to the direction of the static magnetic field B0, the phase of the RF signal that is applied to the RF coil 340 may need to change since the rotation direction of the B1 field is opposite to the predetermined rotation direction.

More specifically, the processor 320 may transmit a control signal for changing the phase of the RF signal that is applied to the RF coil 340 by 90°. As shown in FIG. 6, the processor 320 may transmit a control signal for changing the phases of all RF signals that are applied to the RF coil 340 by 90°. That is, the processor 320 may change the phase of an RF signal having a phase of 0° to 90°, and the phase of an RF signal having a phase of 90° to 0°, among a plurality of RF signals that are applied to the RF coil 340.

The switch 330 may change the phase of the RF signal that is applied to the RF coil 340, based on the control signal transmitted from the processor 320. The switch 330 may be one of various types of switches, such as a T/R switch. However, the switch 330 may be any type of switch as long as it can change the phase of an input signal.

Referring to FIG. 9, when the rotation direction, for example a clockwise direction, of the B1 field formed in the coil apparatus 300c with respect to the direction of the static magnetic field B0 matches with the predetermined rotation direction, the processor 320 may determine that the coil apparatus 300c is positioned in the predetermined direction with respect to the direction of the static magnetic field B0.

Accordingly, the processor 320 may transmit a control signal for not changing the phase of an RF signal that is applied from the RF applier 140 to the RF coil 340, and the switch 330 may not change the phase of the RF signal that is applied to the RF coil 340, based on the control signal from the processor 320.

Referring to FIG. 10, when the rotation direction, for example a counterclockwise direction, of the B1 field formed in the coil apparatus 300c with respect to the direction of the static magnetic field B0 is opposite to the predetermined rotation direction, the processor 320 may determine that the coil apparatus 300c is positioned in the direction that is opposite to the predetermined direction with respect to the direction of the static magnetic field B0.

Accordingly, the processor 320 may transmit a control signal for changing the phase of an RF signal that is applied from the RF applier 140 to the RF coil 340 by 90°, and the switch 330 may change the phase of the RF signal that is applied to the RF coil 340 by 90°, based on the control signal from the processor 320.

That is, as shown in FIG. 10, the switch 330 may change the phase of an RF signal having a phase of 0° to 90°, and the phase of an RF signal having a phase of 90° to 0°, among a plurality of RF signals that are applied to the RF coil 340, and the rotation direction of the B1 field formed in the coil apparatus 300c may change to the clockwise direction by the changed RF signals.

Accordingly, when the coil apparatus 300c is positioned in the direction that is opposite to the predetermined direction with respect to the direction of the static magnetic field B0, the rotation direction of the B1 field may change by changing the phase of the RF signal through the switch 330, so that a MR signal may be acquired.

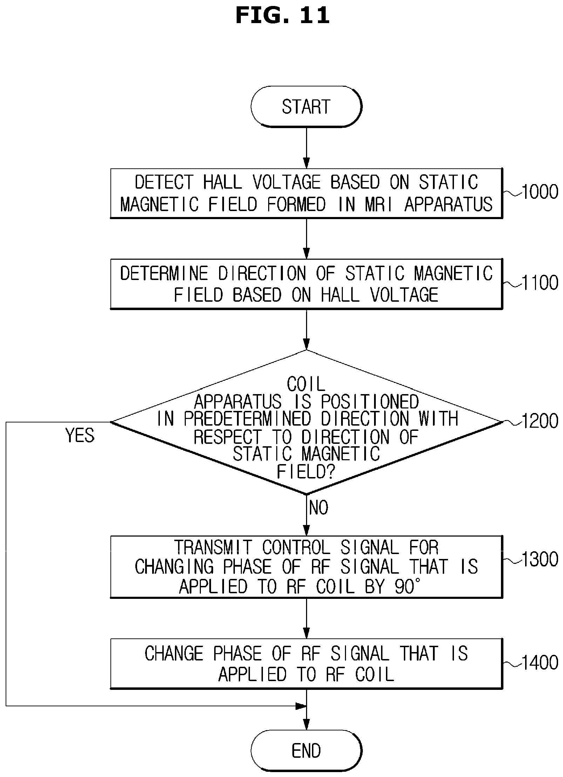

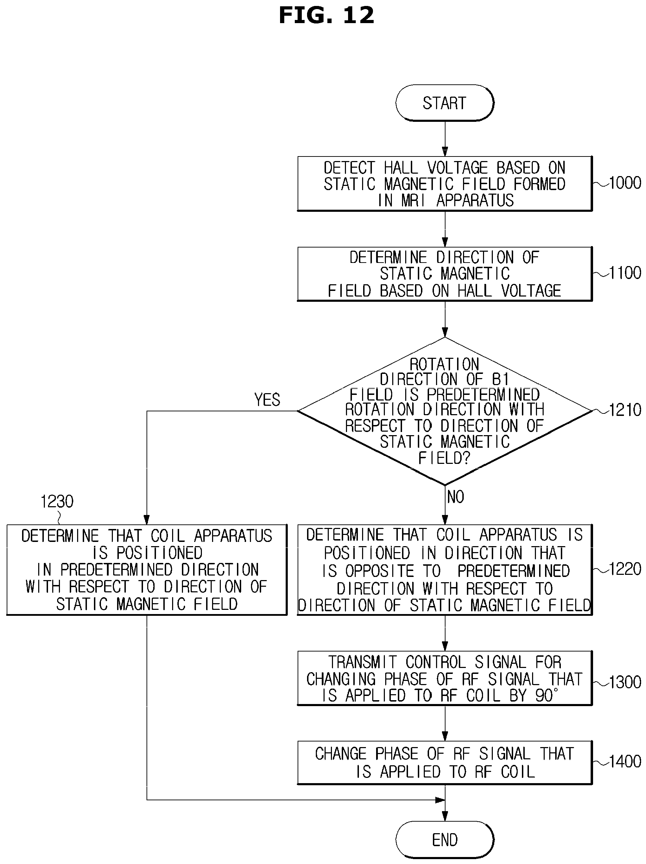

FIGS. 11 and 12 are flow charts illustrating a coil apparatus controlling method according to an embodiment.

Referring to FIG. 11, the sensor 310 may detect a static magnetic field formed in the MRI apparatus 1, and according to an embodiment, the sensor 310 may detect a hall voltage based on a static magnetic field formed in the MRI apparatus 1, in operation 1000. A principle by which the sensor 310 detects a hall voltage has been described above with reference to FIG. 8, and accordingly, overlapping descriptions thereof are omitted.

The processor 320 may determine a direction of the static magnetic field based on the hall voltage detected by the sensor 310, in operation 1100.

That is, because the polarity of the hall voltage detected by the sensor 310 depends on a direction of a potential difference generated based on the static magnetic field formed in the MRI apparatus 1, the processor 320 may determine a direction of the static magnetic field based on the polarity of the hall voltage detected by the sensor 310.

The processor 320 may determine whether the coil apparatus 300 is positioned in a predetermined direction with respect to the determined direction of the static magnetic field, in operation 1200. More specifically, the processor 320 may determine whether a rotation direction of a B1 field formed in the coil apparatus 300 is a predetermined rotation direction with respect to the direction of the static magnetic field, in operation 1210.

When the processor 320 determines that the rotation direction of the B1 field is opposite to the predetermined rotation direction, the processor 320 may determine that the coil apparatus 300 is positioned in a direction that is opposite to the predetermined direction with respect to the direction of the static magnetic field, in operation 1220.

Also, when the processor 320 determines that the rotation direction of the B1 field is the predetermined rotation direction, the processor 320 may determine that the coil apparatus 300 is positioned in the predetermined direction with respect to the direction of the static magnetic field, in operation 1230.

When the processor 320 determines that the coil apparatus 300 is positioned in a direction that is opposite to the predetermined direction, the processor 320 may transmit a control signal for changing a phase of an RF signal that is applied to the RF coil 340 by 90°, in operation 1300. The switch 330 may change the phase of the RF signal that is applied to the RF coil 340 by 90°, based on the control signal from the processor 320, in operation 1400.

Hereinafter, technical features of the coil apparatus 300 according to an embodiment, and a case of applying the coil apparatus control method to the components included in the MRI apparatus 1 will be described.

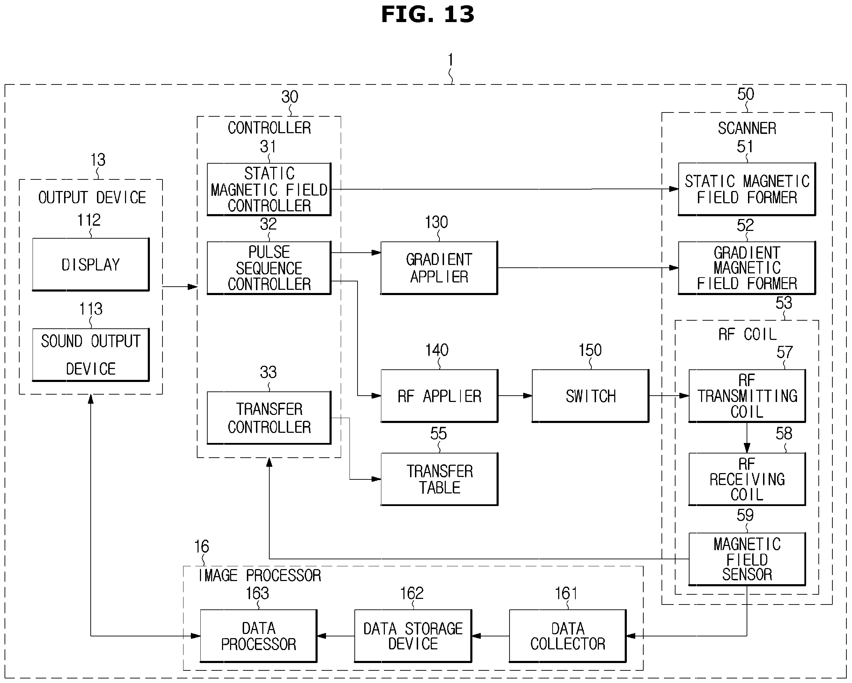

FIG. 13 is a block diagram showing a control flow of a MRI apparatus according to an embodiment.

Referring to FIG. 13, the MRI apparatus 1 according to an embodiment may include the scanner 50 for forming a magnetic field and generating a resonance phenomenon for atomic nuclei, the controller 30 for controlling operations of the scanner 50, determining a direction in which the RF coil portion 53 is positioned, and transmitting a control signal for changing a phase of an RF signal that is applied to the RF coil portion 53, the image processor 16 for generating a MR image based on an echo signal generated from the atomic nuclei, that is, a MR signal, the RF transmitting coil 57 for irradiating an RF signal onto an object, the RF receiving coil 58 for receiving a MR signal generated by the scanner 50 of the MRI apparatus 1 and transferring the MR signal to the image processor 16, a magnetic field sensor 59 for detecting a hall voltage based on a static magnetic field formed by the static magnetic field generator 51, and a switch 150 for changing a phase of an RF signal that is applied to the RF coil portion 53.

The scanner 50 may include the static magnetic field generator 51 for forming a static magnetic field in the inside space, and the gradient magnetic field generator 52 for generating a gradient in a static magnetic field to form a gradient magnetic field. That is, when an object is located in the inside space of the scanner 50, a static magnetic field, a gradient magnetic field, and an RF pulse may be applied to the object. Atomic nuclei constructing the object may be excited by the applied RF pulse, and accordingly, an echo signal may be generated from the object.

The RF receiving coil 58 may receive an RF signal (that is, a MR signal) emitted from the excited atomic nuclei. More specifically, the RF receiving coil 58 may transmit an RF signal of the same frequency as that of precession to the object toward atomic nuclei that perform the precession, then stop transmitting the RF signal to the object, and receive a MR signal emitted from the object.

Generally, the RF receiving coil 58 may be a type fixed on a gantry or a type removably attached on a gantry. When the RF receiving coil 58 is a type removably attached on a gantry, the RF receiving coil 58 may include an RF coil for a part of the object, such as a head coil, a chest RF coil, a leg RF coil, a neck RF coil, a shoulder RF coil, a wrist RF coil, and an ankle RF coil, as described above with reference to FIGS. 2 to 4.

Meanwhile, the RF coil portion 53 according to the present disclosure may include the magnetic field sensor 59. The magnetic field sensor 59 may be a sensor that detects a magnetic field applied by the scanner 50.

More specifically, for example, the magnetic field sensor 59 may detect a hall voltage based on a static magnetic field formed by the static magnetic field generator 51, and transfer the hall voltage to the controller 30. The magnetic field sensor 59 may be, for example, a hall sensor that detects a magnetic field based on the Hall effect.

The magnetic field sensor 59 may be attached on the inner or outer surface of the RF coil portion 53. That is, the magnetic field sensor 59 may be attached on the RF transmitting coil 57 or the RF receiving coil 58 of the RF coil portion 53, or may be an embedded type. Hereinafter, for convenience of description, the magnetic field sensor 59 is assumed to be a hall sensor attached on the RF transmitting coil 57.

The controller 30 may include a static magnetic field controller 31 for controlling an intensity and direction of a static magnetic field formed by the static magnetic field generator 51, a pulse sequence controller 32 for designing a pulse sequence and controlling the gradient magnetic field generator 52 and the RF transmitting coil 57 according to the pulse sequence, and a transfer controller 33 for controlling the transfer table 55 for transferring the object.

The transfer controller 33 may control the transfer table 55 on which the object lies, according to an input signal that a user inputs through a control console.

Meanwhile, the controller 30 may be a processor for controlling overall operations of the MRI apparatus 1. In the current embodiment, the configuration of the controller 30 is divided to the static magnetic field controller 31, the pulse sequence controller 32, and the transfer controller 33, for convenience of description, however, the static magnetic field controller 31, the pulse sequence controller 32, and the transfer controller 33 may be integrated into a single System On Chip (SOC) in hardware.

Also, the controller 30 may be implemented with a memory that stores algorithms for controlling operations of components in the MRI apparatus 1, and data for programs embodying the algorithms, and a processor that performs the above-described operations using the data stored in the memory. The memory and the processor may be implemented as separate chips or a single chip.

The MRI apparatus 1 may include a gradient applier 130 for applying a gradient signal to the gradient magnetic field generator 52, and an RF applier 140 for applying an RF signal to the RF transmitting coil 57. The gradient applier 130 and the RF applier 140 may adjust a gradient magnetic field formed in the inside space of the scanner 50 and an RF applied to atomic nuclei through the control of the pulse sequence controller 32.

The image processor 16 may include a data collector 161 for receiving data for a spin echo signal, that is, a MR signal generated from atomic nuclei, and processing the data to create a MR image, a data storage device 162 for storing the data received by the data collector 161, and a data processor 163 for processing the stored data to create a MR image.

Also, the MRI apparatus 1 may include a user controller for receiving a control command for overall operations of the MRI apparatus 1 from a user. Particularly, the MRI apparatus 1 may receive a command for a scan sequence from a user, and generate a pulse sequence according to the command.

The user controller may include a control console for enabling the user to control the system, a display 112 for displaying a control state and displaying an image created by the image processor 16 to enable the user to diagnose an object's health state, and a sound output device 113 for transferring information to the user.

The display 112 may display an image created by the image processor 16, and display an output screen related to a control of the MRI apparatus 1.

The coil apparatus 300 and the control method described above with reference to FIGS. 1 to 12 may be applied in the same manner to the MRI apparatus 1.

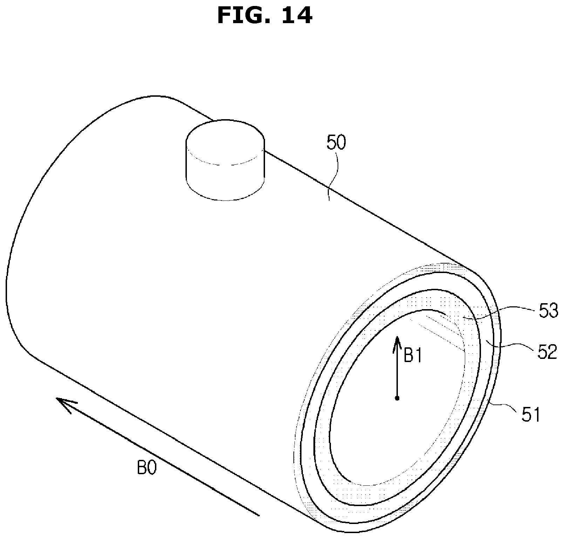

FIG. 14 shows a structure of a scanner according to an embodiment.

Referring to FIG. 14, the MRI apparatus 1 may include the static magnetic field generator 51 for forming a static magnetic field in the inside space of the scanner 50, and the RF coil portion 53 for irradiating an RF signal onto an object.

The RF coil portion 53 shown in FIG. 14 may correspond to the internal structure of the coil apparatus 300 described above with reference to FIG. 6.

That is, when an RF signal is applied to the RF coil portion 53, a B1 field may be formed in the cavity of the RF coil portion 53 in a direction that is perpendicular to a static magnetic field B0 formed in the MRI apparatus 1. Also, by the RF signal applied to the RF coil portion 53, the B1 field may rotate, as described above with reference to FIG. 6.

The RF signal applied to the RF coil portion 53 may be applied to the RF transmitting coil 57 of the RF coil portion 53 through the RF applier 140. At this time, a plurality of RF signals that are applied from the MRI apparatus 1 to the RF coil portion 53 may be applied to the RF transmitting coil 57 with phase differences of 90°.

A rotation direction of the B1 field may change according to a phase of an RF signal that is applied to the RF coil portion 53. The quality of a MR image according to a MR signal may vary depending on the rotation direction of the B1 field.

The MRI apparatus 1 according to an embodiment may determine a direction of a static magnetic field formed in the MRI apparatus 1 and change a phase of an RF signal that is applied to the RF coil portion 53 to thereby operate the RF coil portion 53 normally in a magnetic field formed in the MRI apparatus 1 regardless of a position of the RF coil portion 53.

More specifically, the magnetic field sensor 59 according to an embodiment may detect a hall voltage based on a static magnetic field formed in the MRI apparatus 1, and transfer the hall voltage to the controller 30.

The controller 30 may determine a direction of the static magnetic field formed in the MRI apparatus 1 based on the hall voltage detected by the magnetic field sensor 59. That is, because the polarity of the hall voltage detected by the magnetic field sensor 59 depends on a direction of a potential difference generated based on the static magnetic field formed in the MRI apparatus 1, the controller 30 may determine a direction of the static magnetic field based on the polarity of the hall voltage detected by the magnetic field sensor 59. This operation has been described above with reference to FIG. 8, and accordingly, overlapping descriptions thereof are omitted.

The controller 30 may determine whether the RF coil portion 53 is positioned in a predetermined direction with respect to the determined direction of the static magnetic field. That is, the controller 30 may determine whether the RF coil portion 53 is positioned in a predetermined direction, based on a rotation direction of a B1 field formed in a direction that is perpendicular to the static magnetic field B by an RF signal applied to the RF coil portion 53.

As described above with reference to FIGS. 5 and 6, the rotation direction of the B1 field may change according to a phase of an RF signal that is applied to the RF coil portion 53, and when the RF coil portion 53 is positioned in the predetermined direction with respect to the direction of the static magnetic field B0, a MR image acquired by the rotating B1 field may have high quality.

Accordingly, the controller 30 may determine whether the rotation direction of the B1 field is the predetermined rotation direction with respect to the direction of the static magnetic field B0, and when the controller 30 determines that the rotation direction of the B1 field is a direction that is opposite to the predetermined rotation direction, the controller 30 may determine that the RF coil portion 53 is positioned in a direction that is opposite to the predetermined direction with respect to the direction of the static magnetic field B0.

Also, when the controller 30 determines that the rotation direction of the B1 field is the predetermined rotation direction, the controller 30 may determine that the RF coil portion 53 is positioned in the predetermined direction with respect to the direction of the static magnetic field B0.

When the controller 30 determines that the RF coil portion 53 is positioned in the direction that is opposite to the predetermined direction with respect to the direction of the static magnetic field B0, the controller 30 may transmit a control signal for changing a phase of an RF signal that is applied to the RF coil portion 53.

That is, when the RF coil portion 53 is positioned in the direction that is opposite to the predetermined direction with respect to the direction of the static magnetic field B0, the phase of the RF signal that is applied to the RF coil portion 53 may need to change since the rotation direction of the B1 field is opposite to the predetermined rotation direction.

More specifically, the controller 30 may transmit a control signal for changing the phase of the RF signal that is applied to the RF coil portion 53 by 90°. As shown in FIG. 10, the controller 30 may transmit a control signal for changing the phases of all RF signals that are applied to the RF coil portion 53 by 90°. That is, the controller 30 may change the phase of an RF signal having a phase of 0° to 90°, and the phase of an RF signal having a phase of 90° to 0°, among a plurality of RF signals that are applied to the RF coil portion 53.

The switch 150 may change the phase of the RF signal that is applied to the RF coil portion 53 by 90°, based on the control signal from the controller 30. That is, the switch 150 may change the phase of an RF signal having a phase of 0° to 90°, and the phase of an RF signal having a phase of 90° to 0°, among a plurality of RF signals that are applied to the RF coil portion 53, so that the rotation direction of the B1 field formed in the RF coil portion 53 may change to the opposite direction by the changed RF signals.

Accordingly, when the RF coil portion 53 is positioned in the direction that is opposite to the predetermined direction with respect to the direction of the static magnetic field B0, the rotation direction of the B1 field may change by changing the phase of an RF signal through the switch 150, so that a MR signal of high quality may be acquired.

Therefore, by determining a direction of a static magnetic field formed in the MRI apparatus and changing a phase of an RF signal that is applied to the RF coil, the RF coil may operate normally in a magnetic field formed in the MRI apparatus regardless of its position. Accordingly, it may be possible to reduce time and cost required for adjusting a wrong position of the RF coil, and to prevent an unnecessary waste of scan time.

Meanwhile, the disclosed embodiments may be implemented in the form of a recording medium that stores instructions executable by a computer. The instructions may be stored in the form of program codes, and when executed by a processor, the instructions may create a program module to perform operations of the disclosed embodiments. The recording medium may be implemented as a computer-readable recording medium.

The computer-readable recording medium may include all kinds of recording media storing instructions that can be interpreted by a computer. For example, the computer-readable recording medium may be ROM, RAM, a magnetic tape, a magnetic disc, a flash memory, an optical data storage device, etc.

Although a few embodiments have been shown and described, it would be appreciated by those skilled in the art that changes may be made in these embodiments without departing from the principles and spirit of the disclosure, the scope of which is defined in the claims and their equivalents.

## Claims

1. A coil apparatus comprising:
a Radio Frequency (RF) coil configured to transmit an RF signal onto an object;
a sensor configured to detect a static magnetic field formed in a magnetic Resonance Imaging (MRI) apparatus;
a processor configured to determine a direction of the static magnetic field based on the detected static magnetic field, to determine whether the coil apparatus is positioned in a predetermined direction with respect to the determined direction of the static magnetic field, and to transmit a control signal for changing a phase of an RF signal that is applied to the RF coil, based on a position of the coil apparatus; and
a switch configured to change the phase of the RF signal that is applied to the RF coil, based on the control signal.

2. The coil apparatus according to claim 1, wherein when the processor determines that the coil apparatus is positioned in a direction that is opposite to the predetermined direction, the processor transmits the control signal for changing the phase of the RF signal, and
when the processor determines that the coil apparatus is positioned in the predetermined direction, the processor transmits a control signal for not changing the phase of the RF signal.

3. The coil apparatus according to claim 1 or 2, wherein when the processor determines that the coil apparatus is positioned in a direction that is opposite to the predetermined direction, the processor transmits the control signal for changing the phase of the RF signal that is applied to the RF coil by 90°.

4. The coil apparatus according to any one of the previous claims, wherein the processor determines whether the coil apparatus is positioned in the predetermined direction based on a rotation direction of a B1 field formed in a direction that is perpendicular to the static magnetic field by the RF signal that is applied to the RF coil.

5. The coil apparatus according to claim 4, wherein the processor determines whether the rotation direction of the B1 field is a predetermined rotation direction with respect to the determined direction of the static magnetic field, and when the processor determines that the rotation direction of the B1 field is a direction that is opposite to the predetermined rotation direction, the processor determines that the coil apparatus is positioned in a direction that is opposite to the predetermined direction.

6. The coil apparatus according to any one of the previous claims, wherein the sensor detects a hall voltage based on the static magnetic field formed in the MRI apparatus.

7. The coil apparatus according to claim 6, wherein the processor determines the direction of the static magnetic field based on a polarity of the detected hall voltage, and
the polarity of the hall voltage changes according to a direction of a potential difference generated based on the static magnetic field.

8. The coil apparatus according to any one of the previous claims, wherein the switch changes the phase of the RF signal by 90° according to the control signal, and applies the RF signal to the RF coil.

9. The coil apparatus according to claim 1, further comprising a direct current (DC) conductor, wherein the DC conductor detects a hall voltage based on the static magnetic field formed in the MRI apparatus.

10. The coil apparatus according to any one of the previous claims, wherein the RF coil comprises an RF transmitting coil configured to transmit the RF signal onto the object, and an RF receiving coil configured to receive an excited Magnetic Resonance (MR) signal from the object.

11. The coil apparatus according to any one of the previous claims, wherein the RF coil is a birdcage coil.

12. A magnetic resonance imaging (MRI) apparatus comprising:
a static magnetic field generator configured to form a static magnetic field in the inside space;
a Radio Frequency (RF) coil configured to transmit an RF signal onto an object;
a magnetic field sensor configured to detect the static magnetic field;
a controller configured to determine a direction of the static magnetic field based on the detected static magnetic field, to determine whether the RF coil is positioned in a predetermined direction with respect to the determined direction of the static magnetic field, and to transmit a control signal for changing a phase of an RF signal that is applied to the RF coil, based on the position of the RF coil; and
a switch configured to change the phase of the RF signal that is applied to the RF coil, based on the control signal.

13. The MRI apparatus according to claim 12, wherein the RF coil comprises an RF transmitting coil configured to irradiate the RF signal onto the object, and an RF receiving coil configured to receive an excited Magnetic Resonance (MR) signal from the object,
wherein when the controller determines that the RF transmitting coil is positioned in a direction that is opposite to the predetermined direction, the controller transmits the control signal for changing the phase of the RF signal, and
when the controller determines that the RF transmitting coil is positioned in the predetermined direction, the controller transmits a control signal for not changing the phase of the RF signal.

14. The MRI apparatus according to claim 13, wherein when the controller determines that the RF transmitting coil is positioned in a direction that is opposite to the predetermined direction, the controller transmits the control signal for changing the phase of the RF signal that is applied to the RF coil by 90°.

15. The MRI apparatus according to any one of the claims 12-14, wherein the magnetic field sensor detects a hall voltage based on the static magnetic field formed in the MRI apparatus,
wherein the controller determines the direction of the static magnetic field based on a polarity of the detected hall voltage, and
the polarity of the hall voltage changes according to a direction of a potential difference generated based on the static magnetic field.
